# EUROPEAN PATENT APPLICATION

(11) **EP 4 012 749 A1**
(43) Date of publication of application: **15.06.2022**
(21) Application number: 20849436.9
(22) Date of filing: 05.08.2020
(51) Int. Cl.: H01J 65/00, A61L 2/10, B01J 19/12, H01J 61/16, H01J 61/54

(54) **LIGHT IRRADIATION DEVICE**

(30) Priority: 05.08.2019 JP 2019143960; 07.08.2019 JP 2019145557
(71) Applicant: Ushio Denki Kabushiki Kaisha, Tokyo 100-8150 (JP)
(72) Inventor: YAGYU, Hideaki, Tokyo 100-8150 (JP)
(74) Representative: Tomerius, Isabel
(86) International application number: PCT/JP2020/030007
(87) International publication number: WO 2021/025064

(57) **Abstract**

Provided is a light irradiation device equipped with a plurality of excimer lamps, the light irradiation device capable of achieving high startability of each excimer lamp while suppressing an increase in physical strength required for a light extraction surface. The light irradiation device includes a plurality of lamp units in each of which an excimer lamp is accommodated in an accommodation area surrounded by a wall body. Each of the lamp units includes a light extraction surface for extracting light emitted from the excimer lamp to outside; and a light transmissive window for connection for guiding the light emitted from the excimer lamp to the accommodation area provided in another adjacent lamp unit.

## Description

### TECHNICAL FIELD

The present invention relates to a light irradiation device, and especially relates to a light irradiation device including an excimer lamp.

### BACKGROUND ART

Conventionally, an excimer lamp has a problem of a large-size power source for lighting because a high voltage is required when starting lighting in a low temperature state, a dark state, or after a long dormant state. For this problem, Patent Document 1 below proposes a light irradiation device provided with a UV-LED that radiates ultraviolet light of 390 nm as a start assist light source, the light irradiation device that irradiates an excimer lamp with emission light from the UV-LED to assist start-up.

### PRIOR ART DOCUMENT

### PATENT DOCUMENT

Patent Document 1: JP-A-2017-68944

### SUMMARY OF THE INVENTION

### PROBLEMS TO BE SOLVED BY THE INVENTION

There is a case where higher intensity ultraviolet light is required depending on an application of a consumer. Here, as the simplest method of increasing the intensity of the ultraviolet light emitted from a light irradiation device, there may be a method of increasing the number of excimer lamps mounted on a lamp unit or making the excimer lamp larger.

However, the number of excimer lamps mounted on the lamp unit cannot increase unlimitedly. This is because as the number of excimer lamps increases, an accommodation area for accommodating the excimer lamps increases, so that the lamp unit becomes larger. In order to form the accommodation area for accommodating many excimer lamps, a large housing (frame) is required, and it is necessary to accommodate many excimer lamps in this accommodation area. However, in this case, a heavy accommodated item is accommodated in the accommodation area in the large housing, it is necessary to increase physical strength.

Then, the lamp unit is required to be provided with a light extraction surface for extracting light emitted from the excimer lamp accommodated in the accommodation area out of the lamp unit. This light extraction surface needs to be formed of a substance having high transmissivity with respect to light, and is made of a glass material such as quartz, for example. In order to achieve such light extraction surface made of glass material in a large area, it is necessary to increase a thickness in order to secure high strength. As a result, the light irradiation device becomes extremely large and heavy.

Moreover, even if the light extraction surface is formed of the substance having high transmissivity with respect to light, transmittance is less than 100%, and the light is absorbed at a constant ratio with respect to the incident light. Therefore, if the light extraction surface is formed of a thick glass member in order to secure high strength, an amount of light absorbed by the glass member is not negligible, and light extraction efficiency is lowered.

Note that the above-described problem might occur for a similar reason also in a case where the excimer lamp is made larger instead of increasing the number of excimer lamps mounted on the lamp unit.

Furthermore, there also is a problem that, although some excimer lamps do not have so excellent startability depending on an emission wavelength, it is required to achieve high startability of each excimer lamp in a case where a plurality of excimer lamp is mounted on an ultraviolet irradiation device.

In view of the above-described problems, an object of the present invention is to provide a light irradiation device equipped with a plurality of excimer lamps, the light irradiation device capable of achieving high startability of each excimer lamp while suppressing an increase in physical strength required for a light extraction surface.

### MEANS FOR SOLVING THE PROBLEMS

In view of the above-described problems, the present inventor first examined to achieve a light irradiation device having high light intensity by arranging a plurality of lamp units that accommodates an excimer lamp. According to this configuration, the number of excimer lamps accommodated in each lamp unit is small and the lamp unit may be made small, so that physical strength required for each lamp unit may be relatively small. Since a light extraction surface may be formed on each lamp unit, physical strength required for the light extraction surface may also be relatively small.

As described above, since the excimer lamp does not have so excellent startability depending on an emission wavelength, a light source for assisting start-up might be used as disclosed in Patent Document 1 mentioned above. Therefore, the present inventor achieved the present invention after earnest studies about a method to be adopted for increasing the startability of each excimer lamp in the light irradiation device provided with a plurality of lamp units that accommodates the excimer lamp.

A light irradiation device according to the present invention is provided with
a plurality of lamp units in each of which an excimer lamp is accommodated in an accommodation area surrounded by a wall body, in which
each of the lamp units is provided with:
   a light extraction surface for extracting light emitted from the excimer lamp to outside; and
   a light transmissive window for connection for guiding the light emitted from the excimer lamp to the accommodation area provided in another adjacent lamp unit.

Conventionally, the lamp unit that accommodates the excimer lamp is covered with a material not having transmissivity with respect to light except for the light extraction surface for extracting the light (ultraviolet light) emitted from the excimer lamp to outside. This is to prevent the light emitted from the excimer lamp from being emitted to an unintended place. That is, in a case where a plurality of lamp units is arranged to achieve the light irradiation device having high light intensity, it is usually conceivable to arrange the lamp units so that the light extraction surfaces provided on the lamp units are in the same orientation.

Then, it is conceivable to mount a start assist light source on each lamp unit so that the excimer lamp mounted on each lamp unit is surely lit.

However, in a case where the start assist light source is simply mounted on each lamp unit, the start assist light sources as many as the lamp units forming the light irradiation device are required; probability that one start assist light source of all the start assist light sources provided on an entire light irradiation device enters an unlit state increases. For example, assume that probability that one start assist light source becomes unlit within one year after the start assist light source is mounted is 1%. At that time, in a case where the light irradiation device is equipped with four lamp units and each lamp unit is equipped with one start assist light source, the probability that any one of the start assist light sources provided on the light irradiation device becomes unlit is 1 - (1 - 0.01)⁴ = 0.039 (= 4%). That is, the probability that any one of the start assist light sources provided on the light irradiation device becomes unlit is higher than probability that each start assist light source becomes unlit.

In a case where one start assist light sources becomes unlit, the excimer lamp in the lamp unit equipped with this start assist light source is not lit, and as a result, the intensity of the light extracted from the light irradiation device is lowered.

In contrast, according to the above-described light irradiation device, each lamp unit is provided with a light transmissive window for connection for guiding the light emitted from the excimer lamp to the accommodation area of the adjacent lamp unit. As a result, even if the start assist light source mounted on any one of the lamp units becomes unlit, the excimer lamp is irradiated with the emission light from the excimer lamp accommodated in another lamp unit, so that energy is supplied and discharge may be started.

Then, since each lamp unit is provided with the light transmissive window for connection in this manner, it is not required that all the lamp units of the light irradiation device be provided with the start assist light source. That is, if the excimer lamp mounted on at least any one of the lamp units is lit, it is possible to light the excimer lamp mounted on other lamp units by using the light emitted from the excimer lamp. Then, it is possible to sequentially light other excimer lamps by using the emission light from each excimer lamp.

In other words, it is possible that the light irradiation device itself is not equipped with the start assist light source, and light for assisting start-up is applied into the accommodation area of one lamp unit from outside the light irradiation device to light the excimer lamp provided on the lamp unit. In this case, it is possible to light other excimer lamps one after another.

The light transmissive window for connection may be a simple opening or may be formed of a material that transmits the light emitted from the excimer lamp.

The light transmissive windows for connection provided on a pair of adjacent lamp units may be arranged so as to face each other.

The light irradiation device may be provided with a light guide member that connects the light transmissive windows for connection provided on the pair of adjacent lamp units to each other and guides the light emitted from the excimer lamp.

The light irradiation device may be provided with
a start assist light source that is arranged in the accommodation area provided in at least one of the lamp units and emits light for assisting start-up. In this case, the start assist light source may be fixed to the lamp unit.

Moreover, it is possible that each of all the lamp units is provided with the start assist light source. As described above, even with such a configuration, even if one start assist light source becomes unlit, the excimer lamp in the lamp unit equipped with the start assist light source may be lit, so that lowering in light intensity may be avoided.

The start assist light source may be an LED light source.

Furthermore, the light irradiation device may be provided with a start assist light source that is arranged outside the accommodation area and emits light for assisting start-up toward the accommodation area. In this case, the start assist light source may be arranged outside the lamp unit.

The excimer lamp may emit ultraviolet light, a main emission wavelength of which belongs to a first wavelength band of 190-230 nm, and the start assist light source may emit ultraviolet light, a main emission wavelength of which belongs to a second wavelength band of 250-300 nm.

According to such a configuration, the light irradiation device capable of emitting ultraviolet light suitable for photosterilization from the light extraction surface is achieved.

In a case where a luminescent gas sealed in the excimer lamp contains KrCI, the main emission wavelength of the excimer lamp is 222 nm. In a case where the luminescent gas contains KrBr, the main emission wavelength of the excimer lamp is 207 nm. In a case where the luminescent gas contains ArF, the main emission wavelength of the excimer lamp is 193 nm.

These luminescent gases contain halogen gases such as CI, Br, and F. Since halogen has high electronegativity, this has a high electron attraction property. Therefore, it is considered that the halogen attracts electrons required for discharge, so that the discharge is less likely to start as compared with the Xe excimer lamp, for example.

However, according to the above-described configuration, since the emission light from the excimer lamp mounted on the adjacent lamp unit is incident, even if there is no lighting assist light source or the lighting assist light source becomes unlit, the discharge may be started, and the unlit state of the excimer lamp may be avoided.

Note that, in this specification, the "main emission wavelength" indicates a wavelength λi in a wavelength range Z(λi) showing integrated intensity of 40% or larger with respect to the total integrated intensity in an emission spectrum in a case where a wavelength range Z(λ) of ±10 nm with respect to a certain wavelength λ is defined on the emission spectrum. For example, in a light source having an extremely narrow half-value width and showing light intensity only at a specific wavelength such as an excimer lamp in which a luminescent gas containing KrCI, KrBr, and ArF is sealed, a wavelength having the highest relative intensity (main peak wavelength) may be usually made the main emission wavelength.

### EFFECT OF THE INVENTION

According to the present invention, a light irradiation device capable of improving startability of each excimer lamp while being provided with a plurality of lamp units equipped with the excimer lamp is achieved.

### BRIEF DESCRIPTION OF THE DRAWINGS

Fig. 1 is a cross-sectional view schematically illustrating a configuration of one embodiment of a light irradiation device of the present invention.
Fig. 2 is a perspective view schematically illustrating an appearance of the light irradiation device.
Fig. 3 is a perspective view schematically illustrating each lamp unit illustrated in Fig. 2 in a separated state.
Fig. 4 is a schematic plan view of the light irradiation device as seen in an X direction in which some elements are not illustrated.
Fig. 5 is a schematic plan view of an excimer lamp as seen in the X direction.
Fig. 6 is a schematic plan view of the excimer lamp as seen in a Z direction.
Fig. 7 is an example of a spectrum of ultraviolet light L1 emitted from the excimer lamp in a case where a luminescent gas contains KrCl.
Fig. 8 is a view schematically illustrating a mode of travel of ultraviolet lights (L1 and L2) emitted from the excimer lamp and a start assist light source, respectively.
Fig. 9 is a view illustrating a configuration of the light irradiation device in a case where some lamp units are not provided with the start assist light source following Fig. 4.
Fig. 10 is a view illustrating a configuration of the light irradiation device in a case where all the lamp units are not provided with the start assist light source following Fig. 4.
Fig. 11 is a view illustrating a configuration of a light irradiation device of another embodiment in which a light transmissive window for connection is filled with a material film following Fig. 4.
Fig. 12A is a view illustrating a configuration of a light irradiation device of another embodiment in which a light transmissive window for connection is filled with a material film following Fig. 4.
Fig. 12B is a view illustrating a configuration of a light irradiation device of another embodiment in which a light transmissive window for connection is an opening following Fig. 4.
Fig. 13 is a view illustrating a configuration of a light irradiation device of another embodiment provided with four lamp units following Fig. 4.

### MODE FOR CARRYING OUT THE INVENTION

Each embodiment of a light irradiation device according to the present invention is described with reference to the drawings as appropriate. Note that the following drawings are schematically illustrated, and a dimensional ratios in the drawing are not necessarily the same as the actual dimensional ratios. Furthermore, the dimensional ratios in various figures of the drawings are not necessarily the same, either.

The light irradiation device according to the present invention is provided with a plurality of lamp units in which an excimer lamp is accommodated, and a detailed configuration thereof is to be described later.

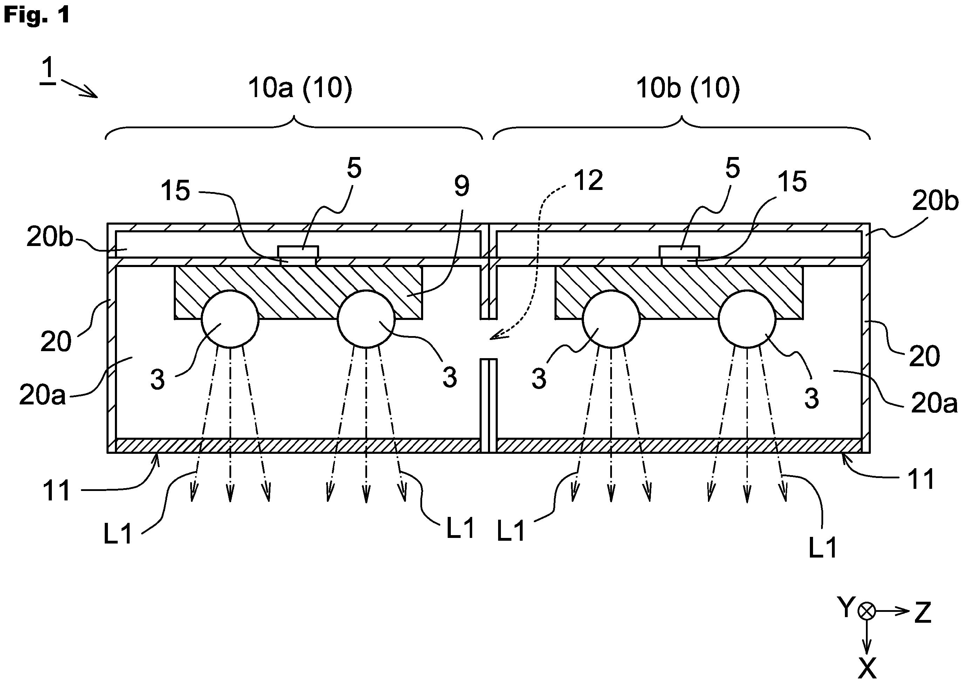

Fig. 1 is a cross-sectional view schematically illustrating a configuration of one embodiment of the light irradiation device. In an example illustrated in Fig. 1, a light irradiation device 1 is provided with two lamp units 10 (10a and 10b). Hereinafter, in a case where the lamp units are distinguished from each other, they are referred to as a "lamp unit 10a" and a "lamp unit 10b", and in a case where they are not distinguished from each other, they are generically referred to as the "lamp unit 10".

The lamp unit 10 is provided with an excimer lamp 3 accommodated in an accommodation area 20a surrounded by a wall body 20. A case where each lamp unit 10 is provided with two excimer lamps (3 and 3) is hereinafter described as an example, but the number of excimer lamps 3 provided on the lamp unit 10 is not limited to two, and may be one or three or larger.

In the light irradiation device 1 illustrated in Fig. 1, each lamp unit 10 has a substantially rectangular parallelepiped shape, and includes a hollow space surrounded by the wall body 20 so that the excimer lamp 3 and the like may be accommodated therein. Then, one surface 11 of the lamp unit 10 forms a light extraction surface for extracting ultraviolet light L1 emitted from the excimer lamp 3 out of the lamp unit 10. This surface 11 (hereinafter referred to as a "light extraction surface 11") is formed of a material showing transmissivity with respect to the ultraviolet light L1 and is made of, for example, quartz glass and the like.

As illustrated in Fig. 1, the light irradiation device 1 is formed by arranging a plurality of lamp units 10, and the light extraction surface 11 made of quartz glass and the like is formed on each lamp unit 10. Therefore, a large-size light extraction surface 11 over an entire light irradiation device 1 is not required, so that bending and deflection are less likely to occur even when the light extraction surface 11 is made thin.

Note that although Fig. 1 illustrates a state in which the lamp unit 10a and the lamp unit 10b are in contact with each other, they may be arranged with a clearance therebetween. The clearance in this case may be set in a range in which the ultraviolet light L1 from the excimer lamp 3 mounted on one lamp unit 10 (10a or 10b) may reach the excimer lamp 3 mounted on the other lamp unit 10 (10b or 10a) to light the same, for example, about a few millimeters to more than ten millimeters.

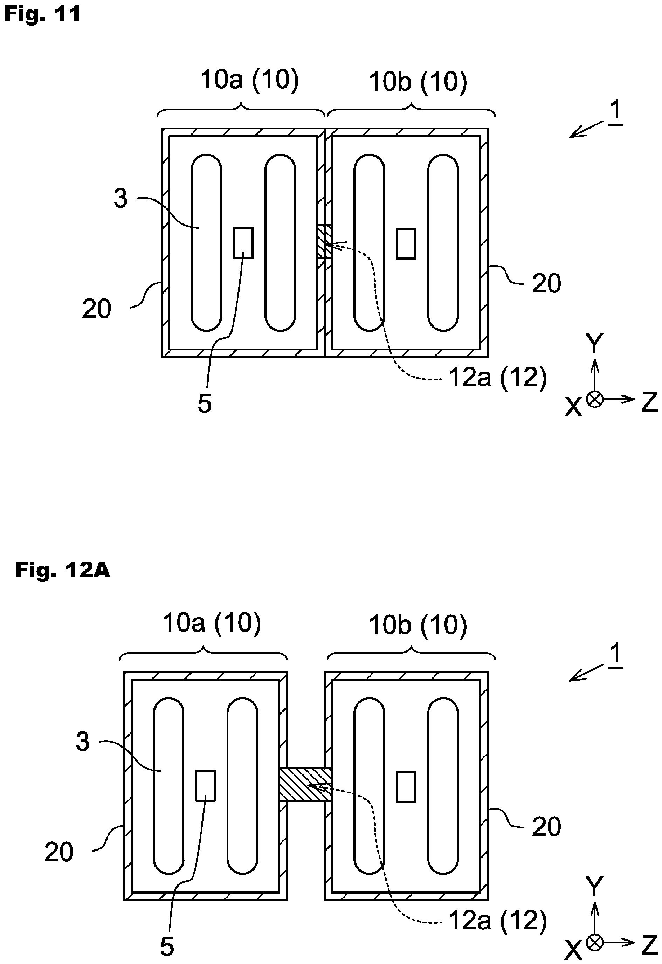

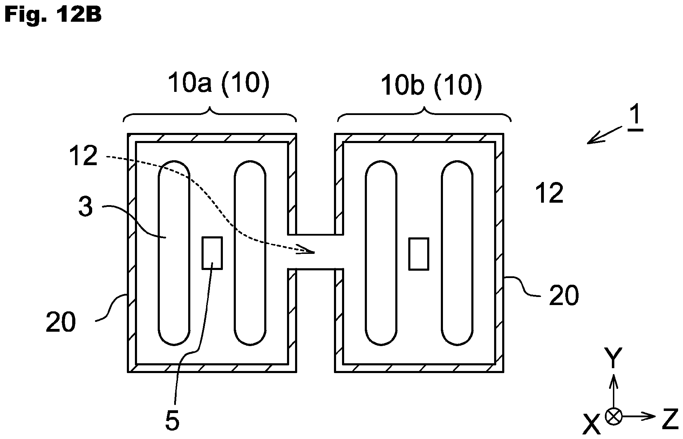

A configuration example of a case where the adjacent lamp units 10 are arranged with the clearance therebetween in this manner is described later with reference to Figs. 12A and 12B.

In the following description, an XYZ coordinate system in which a direction in which the ultraviolet light L1 is extracted from the lamp unit 10 is an X direction and a plane orthogonal to the X direction is a YZ plane is appropriately referred to. Note that a case where the two excimer lamps (3 and 3), a tube axis direction of which is in a Y direction, are arranged apart from each other in a Z direction is illustrated in Fig. 1.

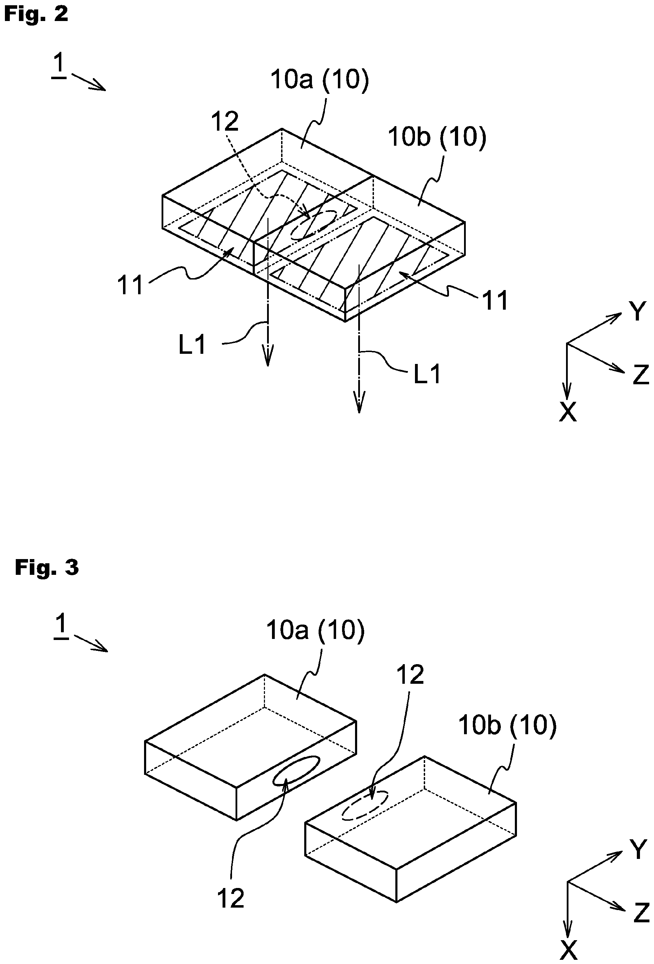

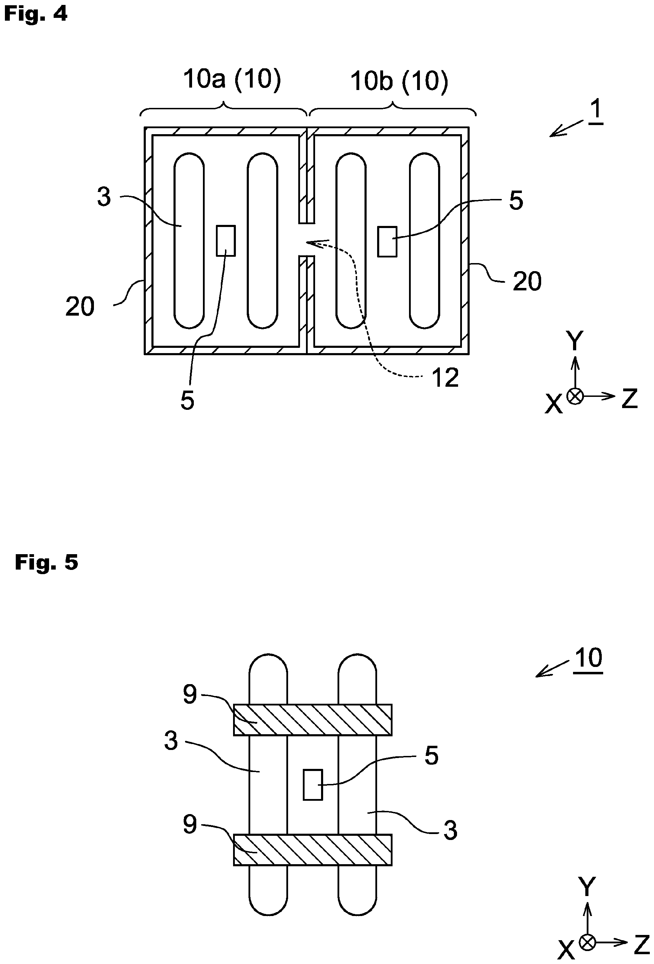

Fig. 2 is a perspective view schematically illustrating an appearance of the light irradiation device 1. Fig. 3 is a perspective view schematically illustrating each lamp unit 10 illustrated in Fig. 2 in a separated state. Fig. 4 is a schematic plan view of the light irradiation device 1 as seen in the X direction. However, in Fig. 4, some elements are not illustrated for convenience of description.

As illustrated in Figs. 1 to 4, each lamp unit 10 is provided with a light transmissive window for connection 12 connected to the adjacent lamp unit. In this embodiment, a case where the light transmissive window for connection 12 is an opening is illustrated. The light transmissive window for connection 12 is provided for guiding a part of the ultraviolet light L1 emitted from the excimer lamp 3 into the adjacent lamp unit 10. In this embodiment, the light transmissive windows for connection 12 provided on a pair of adjacent lamp units 10 are arranged so as to face each other. A function of the light transmissive window for connection 12 is described later in detail.

Fig. 5 is a schematic plan view of the excimer lamps (3 and 3) accommodated in one lamp unit 10 as seen in the X direction. A pair of electrodes (9 and 9) separated in the Y direction are formed on an outer wall of each excimer lamp (3 and 3), the tube axis direction of which is in the Y direction. In Fig. 1, only one electrode 9 of the pair of electrodes (9 and 9) arranged in each lamp unit 10 is illustrated.

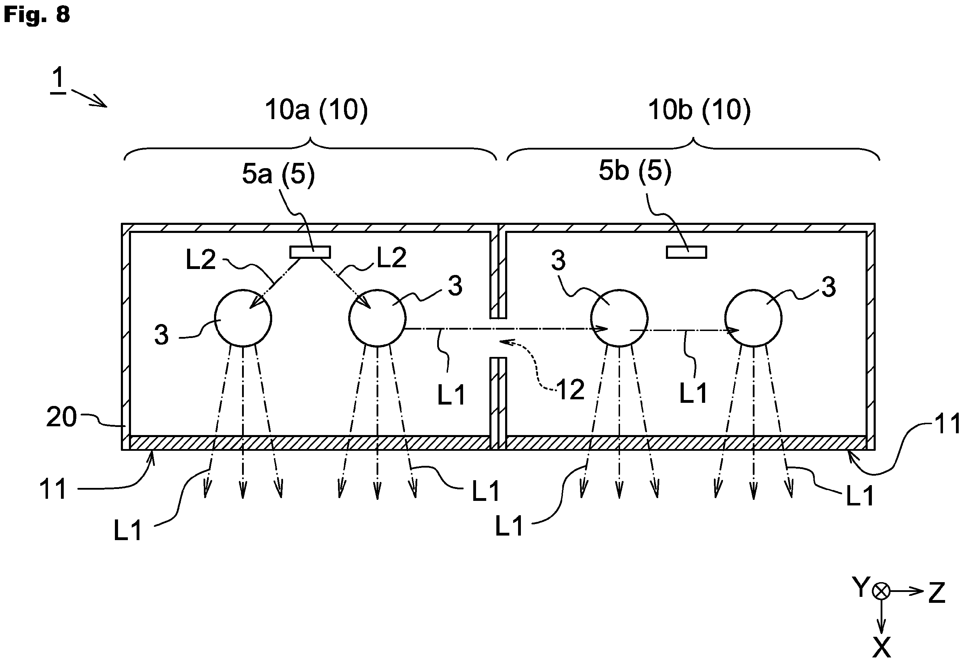

As illustrated in Figs. 1, 4, and 5, the light irradiation device 1 of this embodiment is provided with a start assist light source 5 in each lamp unit 10. In this embodiment, the start assist light source 5 is an LED light source and emits ultraviolet light L2 (refer to Fig. 8), a main emission wavelength of which belongs to a second wavelength band of 250-300 nm. As an example, the start assist light source 5 is the LED light source a peak wavelength of which is 280 nm. In this embodiment, the start assist light source 5 is fixed to the wall body 20 in a space 20b outside the accommodation area 20a in which the excimer lamp 3 is accommodated.

On the lamp unit 10, a light transmissive window for assist light 15 is formed in a position facing a light emission surface of the start assist light source 5 (refer to Fig. 1). The ultraviolet light L2 (refer to Fig. 8) emitted from the start assist light source 5 enters the accommodation area 20a inside the lamp unit 10 through the light transmissive window for assist light 15. The light transmissivewindow for assist light 15 may be formed of a material that transmits the ultraviolet light L2, or may be a simple opening.

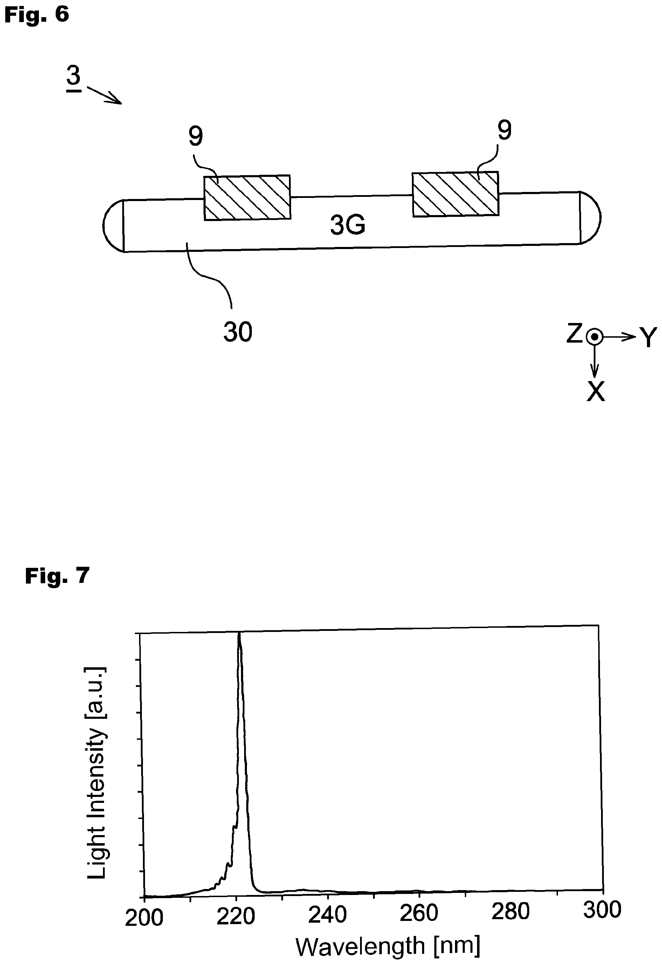

Fig. 6 is a view schematically illustrating a positional relationship between the excimer lamp 3 and the electrodes (9 and 9) and corresponds to a schematic plan view of the excimer lamp 3 as seen in the Z direction.

As described above, the excimer lamp 3 includes a tube body 30, the tube axis direction of which is in the Y direction. The pair of electrodes (9 and 9) are in contact with an outer wall surface of the tube body 30 in positions separated from each other in the Y direction. A luminescent gas 3G is sealed in the tube body 30. When a high-frequency AC voltage of, for example, about 10 kHz to 5 MHz is applied between the pair of electrodes (9 and 9), the voltage is applied to the luminescent gas 3G via the tube body 30. At that time, discharge plasma is generated in a discharge space in which the luminescent gas 3G is sealed, an atom of the luminescent gas 3G is excited to enter an excimer state, and excimer light emission is generated when this atom shifts to a ground state.

A gas type of the luminescent gas 3G is determined according to the wavelength of the ultraviolet light L1intended to be emitted from the light irradiation device 1. For example, the luminescent gas 3G is made of a material that emits the ultraviolet light L1, the main emission wavelength of which belongs to the first wavelength band of 190-230 nm at the time of excimer light emission. At that time, the luminescent gas 3G contains KrCI, KrBr, and ArF. Note that, in addition to the above-described gas types, an inert gas such as argon (Ar) or neon (Ne) may also be mixed.

For example, in a case where the luminescent gas 3G contains KrCI, the excimer lamp 3 emits the ultraviolet light L1, the main emission wavelength of which is in the vicinity of 222 nm. In a case where the luminescent gas 3G contains KrBr, the excimer lamp 3 emits the ultraviolet light L1, the main emission wavelength of which is in the vicinity of 207 nm. In a case where the luminescent gas 3G contains ArF, the excimer lamp 3 emits the ultraviolet light L1, the main emission wavelength of which is in the vicinity of 193 nm. Fig. 7 is an example of a spectrum of the ultraviolet light L1 emitted from the excimer lamp 3 in a case where the luminescent gas 3G contains KrCI.

By the way, in a case where the above-described gas types such as KrCI, KrBr, and ArF are contained as the luminescent gas 3G, the discharge does not start in the tube body 30 even when time elapses only by simply applying the voltage between the pair of electrodes (9 and 9). Therefore, the light irradiation device 1 of this embodiment includes the start assist light source 5. The ultraviolet light L2 emitted from the start assist light source 5 is applied to the excimer lamp 3 in the lamp unit 10 to facilitate the start of the discharge.

Especially, by setting the ultraviolet light L2 emitted from the start assist light source 5 to be the ultraviolet light belonging to the second wavelength band, the main emission wavelength of which is 250-300 nm, startability of the excimer lamp 3 is extremely improved. By irradiating the excimer lamp 3 with the ultraviolet light L2 belonging to the wavelength band while applying the voltage between the pair of electrodes (9 and 9), it is possible to light the excimer lamp 3 within one second, within a few seconds at the latest.

By the way, a case where any one of the start assist light sources 5 mounted on the respective lamp units 10 becomes unlit is assumed. From this point of view, as described above, the light irradiation device 1 is provided with the light transmissive window for connection 12 for connecting the adjacent lamp units 10 to each other. A function of the light transmissive window for connection 12 is described with reference to Fig. 8. Note that, hereinafter, the start assist light source 5 mounted on the lamp unit 10a is referred to as a "start assist light source 5a", and the start assist light source 5 mounted on the lamp unit 10b is referred to as a "start assist light source 5b".

Power supply to each lamp unit 10 is started in order to apply the ultraviolet light L1 from the light irradiation device 1. At that time, while applying a voltage between the pair of electrodes (9 and 9) provided on the excimer lamp 3, lighting control for the start assist light source 5 is performed. Here, a case where the start assist light source 5a is lit but the start assist light source 5b remains unlit due to some trouble is examined.

As schematically illustrated in Fig. 8, the ultraviolet light L2 emitted from the start assist light source 5a is applied to the excimer lamp 3 in the lamp unit 10a to be used for assisting the start-up. As a result, the excimer lamp 3 in the lamp unit 10a starts discharging, and the ultraviolet light L1 is radiated from the light extraction surface 11.

In contrast, a part of the ultraviolet light L1 emitted from the excimer lamp 3 of the lamp unit 10a travels into the adjacent lamp unit 10b through the light transmissive window for connection 12. Then, the ultraviolet light L1 is applied to the excimer lamp 3 of the lamp unit 10b. The ultraviolet light L1 emitted from the excimer lamp 3 has a shorter wavelength and higher energy than those of the ultraviolet light L2 emitted from the start assist light source 5a. Therefore, the ultraviolet light L1 incident from the adjacent lamp unit 10a is used for assisting the start-up of the excimer lamp 3 in the lamp unit 10b.

That is, even in a case where the start assist light source 5b provided on the lamp unit 10b becomes unlit, the ultraviolet light L1 traveling from the adjacent lamp unit 10a may be used as the light for assisting the start-up, so that the excimer lamp 3 in the lamp unit 10b may be lit.

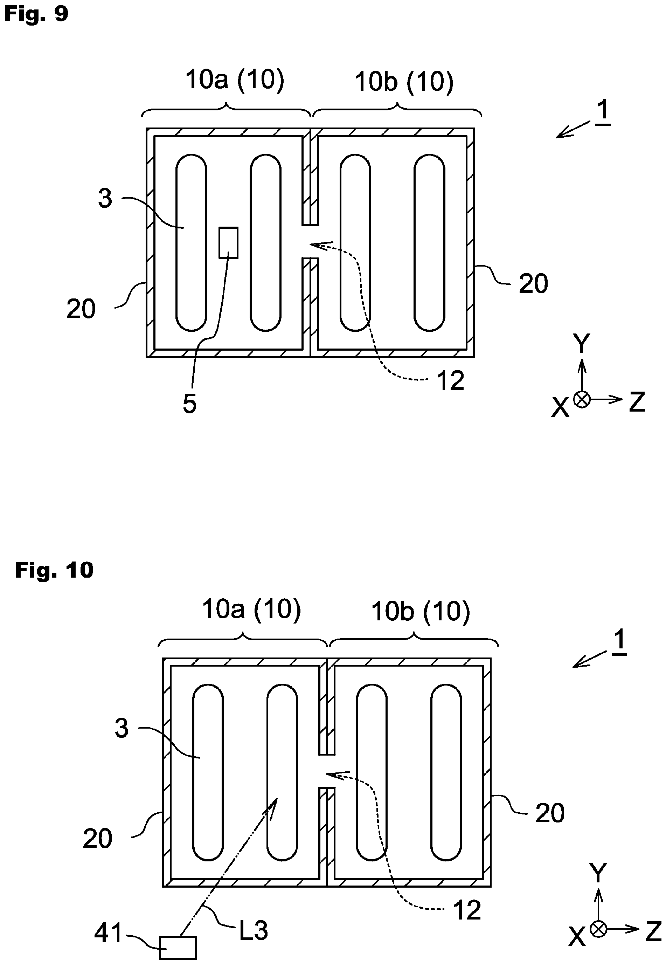

Therefore, it is not always necessary to provide the start assist light source 5 on all of the lamp units 10 provided on the light irradiation device 1. Fig. 9 illustrates a configuration of the light irradiation device 1 in a case where the lamp unit 10a is provided with the start assist light source 5 but the lamp unit 10b is not provided with the start assist light source 5 following Fig. 4.

Moreover, when it is possible to light the excimer lamp 3 in at least one lamp unit 10, the lamp unit 10 does not necessarily have to be provided with the start assist light source 5. Fig. 10 illustrates a configuration of the light irradiation device 1 in a case of lighting the excimer lamp 3 in the lamp unit 10a by irradiating the lamp unit 10a with light L3 for assisting lighting from an external light source 41 arranged outside the lamp unit 10a following Fig. 4. In Fig. 10, once the excimer lamp 3 in the lamp unit 10a is lit, the ultraviolet light L1 traveling from the lamp unit 10a through the light transmissive window for connection 12 serves as the light for assisting the start-up, and the excimer lamp 3 in the lamp unit 10b may be lit.

Note that, in Fig. 10, the external light source 41 may be an LED light source as is the case with the start assist light source 5, an indoor light such as a low-pressure mercury lamp and a fluorescent lamp, or the sun. The light L3 for assisting the start-up emitted from the external light source 41 may be applied from the light extraction surface 11 to the inside of the lamp unit 10, for example.

### [Another embodiment]

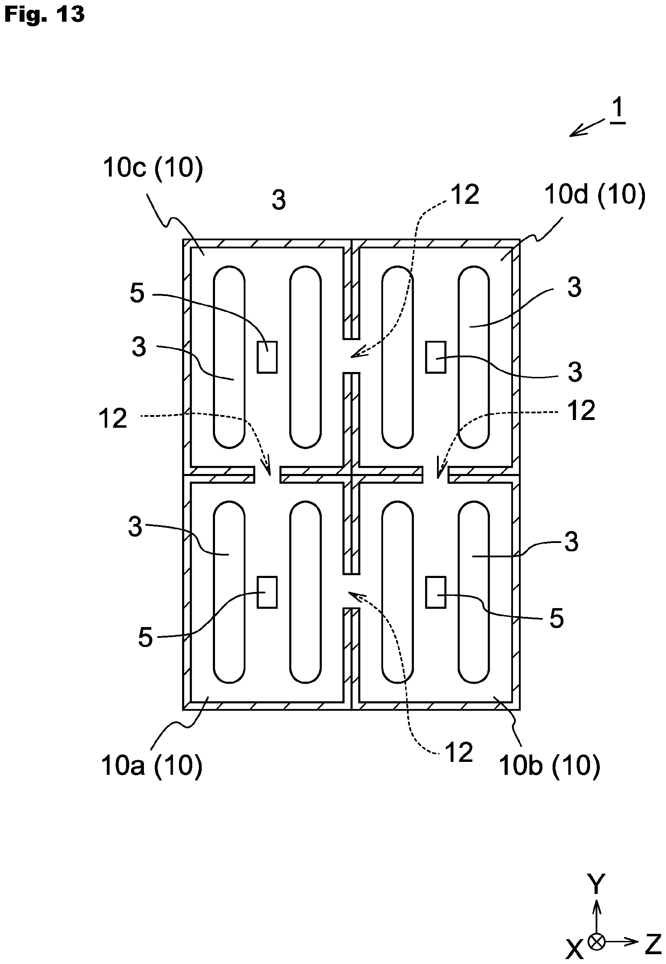

Hereinafter, another embodiment is described.
<1> In the embodiment described above, a case where the light transmissive window for connection 12 is the opening is described. However, the light transmissive window for connection 12 may also be formed of a material 12a, the material having high transmissivity with respect to the ultraviolet light L1 as illustrated in Fig. 11. This material 12a corresponds to a "light guide member".
   Moreover, as illustrated in Fig. 12A, it is also possible that the material 12a is stretched in a direction in which the two lamp units 10 (10a and 10b) are adjacent to each other (here, the Z direction), so that the respective lamp units 10 are separated from each other in a place other than the material film 12a.
<2> Even in a case where the light transmissive window for connection 12 is the opening also, as in Fig. 12A, the adjacent lamp units 10 may be arranged apart from each other in a place where the light transmissive window for connection 12 is not formed (refer to Fig. 12B). In this case, the light transmissive window for connection 12 connecting the two lamp units 10 to each other is formed of a tubular opening area stretching in the Z direction.
<3> Although a case where the light irradiation device 1 is provided with the two lamp units 10 (10a and 10b) is described in the embodiment described above, the number of lamp units 10 is not limited to two, and may be three or larger. Furthermore, the direction in which the plurality of lamp units 10 is arranged is not limited to one direction, and may be a plurality of directions parallel to the light extraction surface 11. Fig. 13 illustrates, as an example, a case where the plurality of lamp units 10 provided on the light irradiation device 1 is arranged such that two lamp units 10 are arranged in the Y direction and two lamp units 10 are arranged in the Z direction.

The light irradiation device 1 illustrated in Fig. 13 is provided with four lamp units 10 (10a, 10b, 10c, and 10d), and each lamp unit 10 accommodates the excimer lamp 3. The lamp unit 10b is arranged adjacent to the lamp unit 10a in the Z direction, the lamp unit 10c is arranged adjacent to the lamp unit 10a in the Y direction, and the lamp unit 10d is arranged adjacent to the lamp unit 10b in the Y direction. The adjacent lamp units 10 are coupled to each other through the light transmissive window for connection 12.

In such a configuration, even if the start assist light source 5 mounted on any one of the lamp units 10 becomes unlit, the ultraviolet light L1 emitted from the excimer lamp 3 mounted on the adjacent lamp unit 10 is incident through the light transmissive window for connection 12, so that this ultraviolet light L1 may be used as the light for assisting the start-up. As a result, a risk that the excimer lamp 3 becomes unlit may be suppressed.

Note that although a case where all the lamp units 10 are provided with the start assist light source 5 is illustrated in Fig. 13, it is not necessarily required that all the lamp units 10 be provided with the start assist light source 5 for the reason similar to that in the above-described embodiment.

<4> In the above-described embodiment, a case where the luminescent gas 3G sealed in the tube body 30 of the excimer lamp 3 contains the gas types of the materials such as KrCI, KrBr, and ArF is described as an example. However, in the present invention, the material of the luminescent gas 3G is not limited.

<5> Although a case where the start assist light source 5 is the LED light source is described in each of the above-described embodiments, the start assist light source 5 may be any light source as long as this is the light source that emits the ultraviolet light L2 and has faster startability than that of the excimer lamp 3. As an example other than the LED light source, the start assist light source 5 may be formed of a low-pressure mercury lamp.

<6> The configuration of the excimer lamp 3 described above with reference to Fig. 5 is merely an example. As an example, the excimer lamp 3 may have a structure in which two tube bodies are provided concentrically and the luminescent gas 3G is sealed between an inner tube and an outer tube (double tube structure). Furthermore, as another example, the excimer lamp 3 may have a structure (single tube structure) in which electrodes are provided inside and outside a single tube body in which the luminescent gas 3G is sealed, or a structure (flat tube structure) in which electrodes are provided on two surfaces facing each other of a tube body having a rectangular surface in which the luminescent gas 3G is sealed.

<7> In the embodiment described above with reference to Fig. 5, a case where the start assist light source 5 is arranged in the position between the pair of electrodes (9 and 9) separated from each other in the Y direction is described. However, in a case where the lamp unit 10 is equipped with the start assist light source 5, the position in which the start assist light source 5 is arranged is any position as long as this is the position in which the excimer lamp 3 accommodated in the lamp unit 10 may be irradiated with the ultraviolet light L2. For example, the start assist light source 5 may be located outside the electrodes (9 and 9) in the Y direction as seen in the X direction. Furthermore, for example, the start assist light source 5 may be arranged in the same space as the accommodation area 20a in which the excimer lamp 3 is accommodated.

### DESCRIPTION OF REFERENCE SIGNS

- 1: Light irradiation device
- 3: Excimer lamp
- 3G: Luminescent gas
- 5 (5a, 5b): Start assist light source
- 9: Electrode
- 10 (10a, 10b, 10c, 10d): Lamp unit
- 11: Light extraction surface
- 12: Light transmissive window for connection
- 15: Light transmissive window for assist light
- 20: Wall body
- 20a: Accommodation area
- 20b: Space outside accommodation area
- 30: Tube body
- 41: External light source

## Claims

1. A light irradiation device comprising
a plurality of lamp units in each of which an excimer lamp is accommodated in an accommodation area surrounded by a wall body,
each of the lamp units including:
a light extraction surface for extracting light emitted from the excimer lamp to outside; and
a light transmissive window for connection for guiding the light emitted from the excimer lamp to the accommodation area provided in another adjacent lamp unit.

2. The light irradiation device according to claim 1, wherein the light transmissive windows for connection provided on a pair of adjacent lamp units are arranged so as to face each other.

3. The light irradiation device according to claim 1 or 2, further comprising
a light guide member that connects the light transmissive windows for connection provided on the pair of adjacent lamp units to each other and guides the light emitted from the excimer lamp.

4. The light irradiation device according to any one of claims 1 to 3, further comprising
a start assist light source that is arranged in the accommodation area provided in at least one of the lamp units and emits light for assisting start-up.

5. The light irradiation device according to claim 4, wherein the start assist light source is fixed to the lamp unit.

6. The light irradiation device according to claim 5, wherein each of all the lamp units is provided with the start assist light source.

7. The light irradiation device according to any one of claims 4 to 6, wherein the start assist light source is an LED light source.

8. The light irradiation device according to any one of claims 1 to 3, further comprising
a start assist light source that is arranged outside the accommodation area and emits light for assisting start-up toward the accommodation area.

9. The light irradiation device according to claim 8, wherein the start assist light source is arranged outside the lamp unit.

10. The light irradiation device according to any one of claims 4 to 9, wherein
the excimer lamp emits ultraviolet light, a main emission wavelength of which belongs to a first wavelength band of 190-230 nm, and
the start assist light source emits ultraviolet light, a main emission wavelength of which belongs to a second wavelength band of 250-300 nm.
